# EUROPEAN PATENT APPLICATION

(11) **EP 4 328 985 A1**
(43) Date of publication of application: **28.02.2024**
(21) Application number: 22191854.3
(22) Date of filing: 24.08.2022
(51) Int. Cl.: H01L 33/38, A61N 5/06, H01L 33/40, H01L 33/00, H01L 33/30, H01L 33/32

(54) **MICRO LED, NEURAL IMPLANT, AND METHOD OF FABRICATING A MICRO LED**

(71) Applicant: Albert-Ludwigs-Universität Freiburg, 79098 Freiburg (DE)
(72) Inventor: KLEIN, Eric, 79106 Freiburg (DE); RUTHER, Patrick, 76119 Karlsruhe (DE)
(74) Representative: Grünecker Patent- und Rechtsanwälte PartG mbB

(57) **Abstract**

The present invention relates to a micro light-emitting diode (LED), a neural implant comprising at least one such micro LED, and to a method of fabricating a micro LED. A micro LED comprises an active side (106), from which radiation is emitted in operation, and a non-emitting side (108), which extends opposite to the active side, and a lateral region (110) which surrounds the micro LED (100) between the active side (106) and the non-emitting side (108), the micro LED (100) further comprising a first semiconductor region (102), a second semiconductor region (104), an optically active region (103) arranged between the first and second semiconductor regions (102, 104), a first contact metallization (112) for electrically contacting the first semiconductor region (102), and a second contact metallization (118) for electrically contacting the second semiconductor region (104), wherein the second contact metallization (118) is electrically connected to the second semiconductor region (104) in a lateral contact region (111) which is part of the lateral region (110).

## Description

The present invention relates to a micro light-emitting diode (LED), a neural implant comprising such a thin-film light-emitting diode (micro LED), and to a method of fabricating the micro LED.

Micro LEDs are a prerequisite for a broad variety of high-resolution displays. The micro LED's light emission and direction in that context strongly correlates with the dimensions and the placement of the required electrical contacts which often block light emission. With conventional micro LEDs, one can distinguish between dual-sided and single-sided interconnection schemes. In case of the dual-sided micro LEDs, one contact is preferably realized using a highly reflective material, i.e. normally interfacing the highly resistive p-doped side of an LED, while the contact of opposite polarity is either kept as small as possible or realized using a transparent electrode material in order not to obstruct the light emission.

In known dual-sided micro LEDs, as for instance shown in Klein E, Gossler C, Paul O & Ruther P (2018), High-density µLED-based optical cochlear implant with improved thermomechanical behavior. Front Neurosci 12: Article 659 (15pp), the n-contact is laid out to have a circular aperture. In case of the single-sided interconnection scheme, as shown for instance in US 6,573,537 B1 or Klein E, Ulrich CR, Paul O & Ruther P (2021) Stackable Wireless Controller of Single-sided µLED Arrays for Optogenetics in Freely Behaving Animals. In IEEE MEMS Conf. 2022, Tokyo, Japan, pp. 114-117, doi: 10.1109/MEMS51670.2022.9699683, the LED mesa has an etched recess exposing the n-doped material. Due to the low resistivity of the n-doped micro LED material, the contact dimensions can be kept as small as possible. The p-contact is made as large as possible in order to minimize the contact resistance and optimize the current distribution. The differences in the basic layout of both interconnecting schemes is given by the respective processing complexity and the fact that the single-sided scheme is characterized by an asymmetric current distribution within the micro LED mesa and current crowding at the smaller n-contact (this has been circumvented for instance by the vertical chip technology by Hahn B, Galler B & Engl K (2014) Development of high-efficiency and high-power vertical light-emitting diodes. Jpn J Appl Phys 53: 100208, and US 2012/0018763 A1). In addition, any further minimization of the micro LED footprint increases the fabrication complexity in particular of the single-sided approach.

Aside from their application in consumer electronics and automotive industry for car lights, micro LEDs are found for instance in optical implants, preferably realized on flexible substrates, for optogenetic stimulation of the central and peripheral nervous system. The key benefit of micro LED-based implants is the amount of channels and the fairly high lateral resolution which can be achieved, both integrated into very small and flexible implants. Micro LEDs used as light sources allow neural tissue to be optically stimulated with high temporal and spatial resolution. Optogenetic devices are applied in a broad variety of optogenetic studies; a first phase clinical trial is currently on the way.

Known gallium nitride (GaN) LEDs and micro LEDs are typically fabricated using sapphire as an epitaxial growth substrate on which n-doped and p-doped semiconductor layers are deposited, with the optically active layers sandwiched in-between. In case of the dual-sided scheme, the semiconductor layer stack is typically patterned by dry etching after deposition of the p-contact. The micro LEDs are transferred onto a substrate followed by laser lift-off from the sapphire substrate and formation of the n-doped contact. The substrates to which the micro LEDs are bonded have been stiff or flexible, as documented in literature.

In case of the single-sided contact scheme, the semiconductor layer stack is patterned into micro LED mesas with a recessed contact to the n-doped layer. This is followed by deposition of the n- and p- contacts which are tailored in view of their optical reflectivity. Micro LEDs can be transferred either serially by transfer printing (Kim T-I, McCall JG, Jung YH, Huang X, Siuda ER, Li Y, Song J, Song YM, Pao HA, Kim R-H, et al (2013) Injectable, Cellular-Scale Optoelectronics with Applications for Wireless Optogenetics. Science 340: 211-216) and flip-chip bonding (see patent application US 6,573,537 B1).

As an alternative, the semiconductor layer stack can be grown on silicon (Kim K, Voroslakos M, Seymour JP, Wise KD, Buzsáki G & Yoon E (2020) Artifact-free and high- temporal-resolution in vivo opto-electrophysiology with microLED optoelectrodes. Nat Commun 11: 2063; and Scharf R, Tsunematsu T, McAlinden N, Dawson MD, Sakata S & Mathieson K (2016) Depth-specific optogenetic control in vivo with a scalable, high-density µLED neural probe. Sci Rep 6: 28381 (10pp)) using an alternative single-sided interfacing approach.

Examples of state-of-the-art optogenetic probes applying micro LEDs are for instance micro LEDs grown on silicon and interconnected via a common n-contact and individual p-contacts. Light coupling out towards the p-doped side is partially blocked by p-contact; the micro LEDs do not apply any mirror layer to optimize light extraction (see Kim K, Voroslakos M, Seymour JP, Wise KD, Buzsáki G & Yoon E (2020) Artifact-free and high- temporal-resolution in vivo opto-electrophysiology with microLED optoelectrodes. Nat Commun 11: 2063). Further, it is known to transfer micro LED from sapphire to silicon to establish a double-sided connection. The current flows from facet to facet, and light coupling out is partially blocked by the n-contact aperture. Except for the highly reflective p-contact, these micro LEDs do not apply lateral mirrors (Ayub S, David F, Klein E, Borel M, Paul O, Gentet L & Ruther P (2020) Compact optical neural probes with up to 20 integrated thin-film µLEDs applied in acute optogenetic studies. IEEE Trans Biomed Eng 67: 2603-2615).

Klein E, Gossler C, Paul O & Ruther P (2018) High-density µLED-based optical cochlear implant with improved thermomechanical behavior. Front Neurosci 12: 659-659 (15pp); Gossler C, Bierbrauer C, Moser R, Kunzer M, Holc K, Pletschen W, Kohler K, Wagner J, Schwaerzle M, Ruther P, et al (2014) GaN-based micro-LED arrays on flexible substrates for optical cochlear implants. J Phys D: Appl Phys 47: 205401-205401; and US 10,276,631 B2 disclose micro LEDs transferred from the epitaxial sapphire substrate to a polymer substrate using wafer-level bonding and laser-lift off. Except for the highly reflective p-contact, these micro LEDs do not apply lateral mirrors.

Furthermore, transfer-printed, single-sided micro LEDs are known from Kim T-I, McCall JG, Jung YH, Huang X, Siuda ER, Li Y, Song J, Song YM, Pao HA, Kim R-H, et al (2013) Injectable, Cellular-Scale Optoelectronics with Applications for Wireless Optogenetics. Science 340: 211-216, and Shin G, Gomez AM, Al-Hasani R, Jeong YR, Kim J, Xie Z, Banks A, Lee SM, Han SY, Yoo CJ, et al (2017) Flexible Near-Field Wireless Optoelectronics as Subdermal Implants for Broad Applications in Optogenetics. Neuron 93: 509-521.

Klein E, Ulrich CR, Paul O & Ruther P (2021) Stackable Wireless Controller of Single-sided µLED Arrays for Optogenetics in Freely Behaving Animals. In IEEE MEMS Conf. 2022, Tokyo, Japan, pp. 114-117, doi: 10.1109/MEMS51670.2022.9699683, discloses single-sided micro LEDs integrated into a polymer substrate. These micro LEDs are equipped with recessed n-contacts.

There is still a need for a micro LED that has a high radiation output efficiency, can be electrically contacted in a particularly efficient manner, and at least partly avoids the drawbacks of existing technology. Further, there is a need for a method of fabricating such a micro LED and for a neural implant comprising an array of such micro LEDs.

This object is solved by the subject matter of the independent claims. Advantageous examples are the subject matter of the dependent claims.

The present disclosure is based on the idea to electrically contact one of the PN-layers of an LED at a lateral side of the chip (also called LED mesa in the following) and providing the electrical connection to the external circuitry on the same surface of the LED mesa as the electrical connection for the other one of the PN-layers. Thus, by making use not only of the top and/or bottom planes, but also of at least a part of the lateral surface of the LED mesa, the overall active area per micro LED is increased in comparison to the conventional single-sided surface contacts. This effect is especially strong for micro LEDs with a small area, e. g. less than 100×100 µm². Moreover, by providing a highly reflective metallization around most of the PN-layers of the LED mesa, leaving open only the light-emitting active area, stray light which would normally be exiting the micro LED mesa through its sidewalls is reflected back increasing the chance that it exits the micro LED through the desired active side.

Furthermore, by exploiting the entire circumference of the micro LED mesa (e. g. having a contact height of ca. 3 µm, its length being defined by the footprint, for instance 4x50 µm = 200 µm, resulting in a contact area of 600 µm²), a low contact resistance and a homogeneous and highly symmetric current flow in the micro LED mesa can be achieved, avoiding the current crowding at the smaller contact which is characteristic for the single-sided approach of conventional micro LEDs.

In particular, the present disclosure proposes a micro LED comprising an active side, from which radiation is emitted in operation, and a non-emitting side, which extends opposite to the active side, and a lateral region which surrounds the micro LED between the active side and the non-emitting side, the micro LED further comprising a first semiconductor region, a second semiconductor region, an optically active region arranged between the first and second semiconductor region, a first contact metallization for electrically contacting the first semiconductor region, and a second contact metallization for electrically contacting the second semiconductor region. The second contact metallization is electrically connected to the second semiconductor region in the lateral region. In particular, the second contact metallization is electrically connected to the second semiconductor region in a lateral contact region which is part of the lateral region.

According to an advantageous example of the present disclosure, the first semiconductor region is a p-doped region, and the second semiconductor region is an n-doped region. Thus, the light can be coupled out at the n-doped region which is preferable because the p-doped region usually has inferior light conducting characteristics.

According to a further advantageous example of the present disclosure, the first contact metallization comprises a planar electrode arranged on the non-emitting side for electrically contacting the p-doped region. This arrangement has the advantage that the electrode itself can serve as a mirror for the generated radiation, reflecting any stray radiation towards the desired emitting side.

In particular, the n-doped region may comprise n-doped GaN, and the p-doped region may comprise p-doped GaN. GaN is a semiconductor material characterized by a direct bandgap, which enables it to emit light which is being used for the LED industry. The high speed and efficiency of GaN makes it well suited for use in RF devices and also for optogenetic applications. Moreover, GaN is naturally resistant against damage by moisture and radiation. It is of course clear, that any other material combination, which can be used for forming a PN junction which is operable to emit radiation under electric control (a compound junction), is intended to be covered by the present disclosure. The light-emitting diode emits light when it is forward biased. When a voltage is applied across the junction to make it forward biased, current flows as in the case of any PN junction. Holes from the p-type region and electrons from the n-type region enter the junction and recombine. When this occurs energy is released, some of which is in the form of photons of a given wavelength.

The semiconductor material used for the compound junction must be a compound semiconductor. Compound semiconductors including gallium nitride, gallium arsenide, gallium phosphide, and indium phosphide are compound semiconductors and junctions made from these materials emit light. These compound semiconductors are classified by the valence bands their constituents occupy. For gallium nitride, gallium has a valency of three and nitride a valency of five and this is what is termed a group III-V semiconductor and there are a number of other semiconductors that fit this category. In the present disclosure, only GaN is described in more detail as a specific example. Other materials may of course also be used for the micro LED according to the present disclosure. To produce light of a desired wavelength range, the PN junction must be optimized and the correct materials must be chosen. Pure GaN emits light in the blue range, whereas gallium arsenide releases energy in the infra read portion of the spectrum. To bring the light emission into the visible red end of the spectrum aluminium is added to the semiconductor to give aluminium gallium arsenide (AIGaAs). Phosphorus can also be added to give red light. For other colors, other materials are used. For example, gallium phosphide gives green light and aluminium indium gallium phosphide is used for yellow and orange light. Most LEDs use gallium-based semiconductors.

According to a further advantageous example of the present disclosure, the second contact metallization at least partly covers the non-emitting side and the lateral region. Thereby, a closed reflective casing can be provided around the LED mesa, leaving a radiation transparent region only in the active region from which the radiation is intended to be coupled out.

Advantageously, the first contact metallization and/or the second contact metallization may comprise silver or a silver alloy. Other metals with high electrical conductivity and high optical reflectivity may of course also be used.

For protecting the LED mesa against external influences, the micro LED may further comprise an outer passivation layer that covers the non-emitting side and the lateral region, the outer passivation layer being provided with at least one first contact hole through which the first contact metallization is accessible, and with at least one second contact hole through which the second contact metallization is accessible.

The present disclosure further relates to a neural implant comprising at least one micro LED, and further comprising an at least two-layered carrier substrate, comprising a carrier layer and a first flexible polymer layer. For instance, an optogenetic device as described in detail in Klein E, Ulrich CR, Paul O & Ruther P (2021) Stackable Wireless Controller of Single-sided µLED Arrays for Optogenetics in Freely Behaving Animals. In IEEE MEMS Conf. 2022, Tokyo, Japan, pp. 114-117, doi: 10.1109/MEMS51670.2022.9699683, can advantageously be equipped with micro LEDs according to the present disclosure. Thereby, a particularly efficient and safe neural implant can be realized.

Finally, the present disclosure relates to a method of fabricating a micro LED, the method comprising the following steps:
depositing an optically active layer on a working substrate, the optically active layer comprising a first semiconductor region, a second semiconductor region which is in contact with the working substrate, and an optically active region arranged between the first and second semiconductor region;
depositing and structuring a first contact metallization to contact the first semiconductor region;
structuring the first semiconductor region and at least a part of the second semiconductor region to expose a sidewall of the first semiconductor region, a sidewall of the optically active region, and a partial sidewall of the second semiconductor region;
depositing an inner passivation layer so that it covers the first contact metallization, the sidewall of the first semiconductor region, the sidewall of the optically active region, and the partial sidewall of the second semiconductor region;
performing an etching step, which exposes a lateral contact region of the second semiconductor region, the lateral contact region not being covered by the inner passivation layer;
depositing and structuring a second contact metallization, so that the second contact metallization is electrically connected to the second semiconductor region in a lateral region comprising the lateral contact region.

As mentioned above, the micro LED fabricated by this manufacturing method has the advantage that by making use not only of the top and bottom planes, but also of at least a part of the lateral surface of the LED mesa, the overall active area per micro LED is increased in comparison to the conventional single-sided surface contacts. Moreover, by providing a highly reflective metallization around most of the PN-layers of the LED mesa, leaving open only the light-emitting active area, stray light which would normally be exiting the micro LED mesa through its sidewalls is reflected back increasing the chance that it exits the micro LED through the desired active side.

Furthermore, by exploiting the entire circumference of the micro LED mesa, a low contact resistance and a homogeneous and highly symmetric current flow in the micro LED mesa can be achieved, avoiding the current crowding at the smaller contact which is characteristic for the single-sided approach of conventional micro LEDs.

According to an advantageous example, the first contact metallization and/or the second contact metallization has in at least a part of the spectral range between 300 nm and 1200 nm a reflectivity of more than 98 % and a transmittance of less than 2 %. Thus, radiation is reflected inside the LED mesa in order to exit with a higher probability at the desired surface.

As mentioned above, passivation layers may be applied for providing protection and/or electrical insulation. For instance, the inner passivation layer and/or the outer passivation layer may comprise a silicon nitride and/or a silicon dioxide layer. Other inert, electrically insulating and preferably transparent materials may of course also be used.

The accompanying drawings are incorporated into the specification and form a part of the specification to illustrate several embodiments of the present invention. These drawings, together with the description serve to explain the principles of the invention. The drawings are merely for the purpose of illustrating the preferred and alternative examples of how the invention can be made and used, and are not to be construed as limiting the invention to only the illustrated and described embodiments. Furthermore, several aspects of the embodiments may form-individually or in different combinations-solutions according to the present invention. The following described embodiments thus can be considered either alone or in an arbitrary combination thereof. Further features and advantages will become apparent from the following more particular description of the various embodiments of the invention, as illustrated in the accompanying drawings, in which like references refer to like elements, and wherein:
- **Fig. 1**: is a schematic cross-sectional view of a micro LED according to an example of the present disclosure;
- **Fig. 2**: is a schematic sectional view along the line II-II' of Fig. 1;
- **Fig. 3**: is a schematic top view of the micro LED of Fig. 1;
- **Fig. 4 to 10**: illustrate the manufacturing steps according to an example of a method of fabricating a micro LED;
- **Fig. 11**: is a schematic perspective view of a micro LED-based neural implant;
- **Fig. 12**: is a detail of Fig. 11, showing the base of the implant and the bonding interface;
- **Fig. 13**: is another detail of Fig. 11, showing a sectional view along the line XIII-XIII' of Fig. 11;
- **Fig. 14**: is another detail of Fig. 11, showing the electrical connection of the micro LED;
- **Fig. 15 to 22**: illustrate the manufacturing steps for fabricating a neural implant using at least one micro LED.

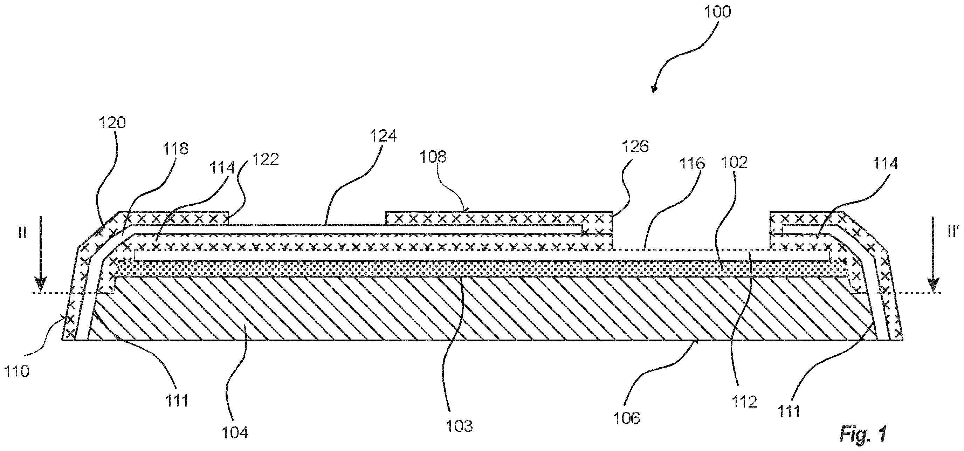

The present disclosure will now be explained in more detail referring to the Figures. Figure 1 shows a schematic cross-sectional view of a micro LED 100 according to an advantageous example. The micro LED 100, also referred to as LED mesa in the following, comprises as a first semiconductor region a p-doped GaN layer 102, a second semiconductor layer formed by an n-doped GaN layer 104, and an optically active region 103, which is arranged between the first semiconductor region 102 and the second semiconductor region 104. The optically active region 103 may e. g. comprise a simple PN junction or a more complex multilayer structure, e. g. quantum wells. It should be noted that the optically active region 103 may have any suitable structure and its particular configuration is not relevant for the present disclosure. In operation, radiation is coupled out on the active side 106, whereas on the non-active side 108 and the lateral region 110 of the micro LED 100, reflective layers block any radiation from being released.

For electrically contacting the p-doped GaN layer 102, a first contact metallization 112 is provided. The first contact metallization 112 may for instance comprise silver or another metal or metal alloy having a low electrical resistance and a high reflectivity for radiation in the wavelength range emitted at the optically active region 103 between the first semiconductor region 102 and the second semiconductor region 104. Advantageously, the first contact metallization 112 covers most of the footprint of the micro LED 100. For protection and electrical insulation, the first contact metallization 112 is covered by an inner passivation layer 114. The first contact metallization 112 is accessible at a first contact region 116, where the inner passivation layer 114 has an opening.

According to the present disclosure, a second contact metallization 118 is provided in the lateral region 110 for electrically contacting the n-doped GaN layer 104 in a lateral contact region 111. The second contact metallization 118 also may for instance comprise silver or another metal or metal alloy having a low electrical resistance and a high reflectivity for radiation in the wavelength range emitted at the optically active region 103 between the first semiconductor region 102 and the second semiconductor region 104. Advantageously, the second contact metallization 118 is deposited in the lateral region 110 around the complete circumference of the micro LED 100, and also covers most of the non-active side 108 of the micro LED 100. Thus, an essentially closed mirror is formed on all surfaces of the LED mesa 100 except the active side 106, and the radiation is emitted on the active side 106 with a higher efficiency.

For providing a final protection and electrical insulation, the micro LED is covered with an outer passivation layer 120. The outer passivation layer 120 may be formed by one or multiple layers of silicon nitride and/or silicon dioxide and/or an organic layer. A second contact hole 122 is provided to give electrically conductive access to a second contact region 124 of the second contact metallization 118. Together with the aforementioned opening in the inner passivation 114, the outer passivation 120 forms a first contact hole 126.

An important aspect of the micro LED design described in this disclosure is given by the fact that the whole surface of the micro LED 100, except for the light exiting facet 106, is coated by a highly reflective metal layer. This metal serves as contact for either the p- and n-doped layers of the micro LED or as a reflective shield blocking stray light from exiting the micro LED into unwanted directions.

As will become apparent from the description further below, in order to realize this metal contact, a wafer-level process is conducted on the epitaxial sapphire growth substrate, which is further used to process the flexible implant around the micro LEDs. In comparison to existing approaches, as described above, the following main advantages are seen. Light which is generated at the optically active region 103 is optimally coupled out in a preferred direction; stray light is reflected into the preferred direction increasing the usable optical power. The contact placement restrictions are more relaxed because positioning the n-contact around the p-contact on the lateral sidewalls of the LED-mesa enables a more homogeneous current path within the micro LED. Furthermore, the area of the n-contact is maximized in comparison to other single-sided approaches enabling an optimized homogeneous current path avoiding current crowding and providing light reflection.

The micro LED 100 has an overall larger contact area for a given micro LED size, reducing process complexity and effort; alternatively, micro LED dimensions can be reduced using the same contact size.

As will be described in more detail below, a single-sided process enables embedding an array of micro LEDs 100 into flexible polymers, thus avoiding wafer-level bonding processes.

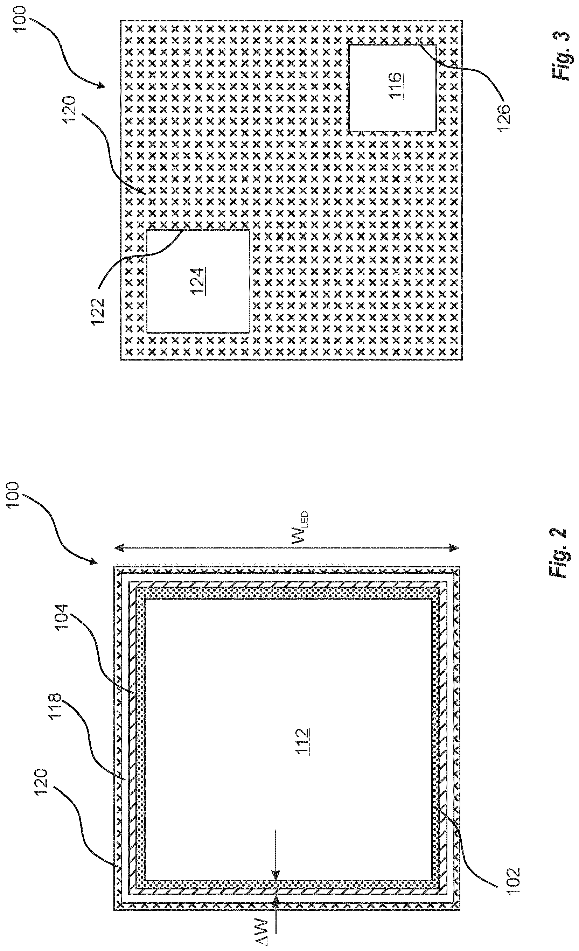

Figure 2 shows a sectional view as seen from the non-active side 108 towards the active side 106 (which can also be referred to as the light-emitting facet) along the line II-II' of Fig. 1. The total width W_{LED} defines the footprint of the micro LED 100. It should be noted that the overlap ΔW is depending on the applied photolithography and does not scale with the lateral dimensions of the micro LED 100.

It can be seen from Fig. 2 that the first contact metallization 112 forms a planar electrode which covers more than two thirds of the total footprint of the micro LED 100. Thus, an effective reflective mirror is provided for ensuring that as much radiation is emitted through the light-emitting facet 106 as possible.

Figure 3 shows a top view onto the non-active side 108 of the micro LED 100 of Fig. 1. As mentioned above, the micro LED 100 according to the present disclosure falls under the category of a single-sided LED, meaning that both the first contact region 116 as well as the second contact region 124 are arranged on the non-active side 108 of the micro LED 100. This allows for a simplified electrical connection of the micro LED 100 in its application environment. Moreover, it allows for the light-emitting facet 106 having the maximum possible area.

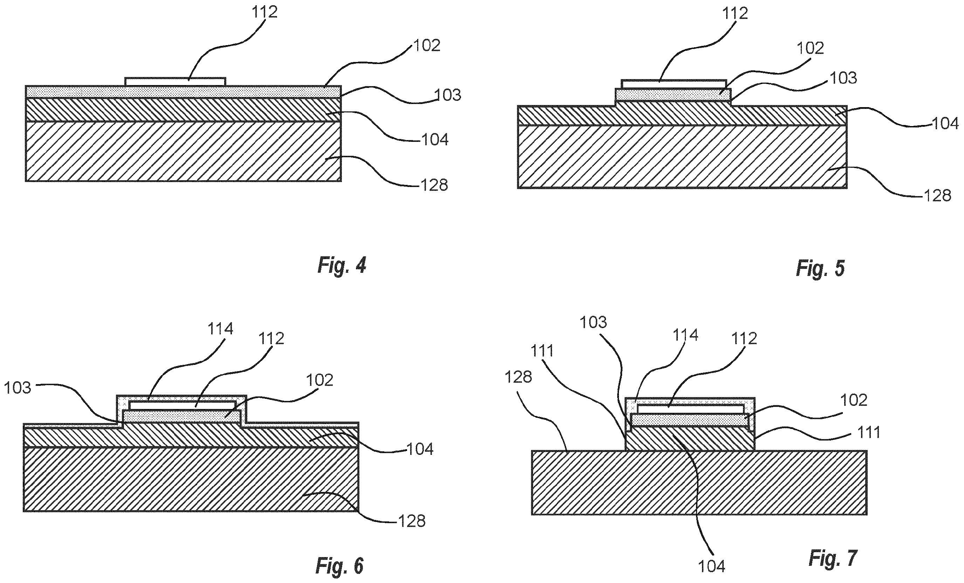

With reference to Fig. 4 to 10, the main steps of an advantageous manufacturing process will be explained for fabricating the micro LED 100, as shown in Fig. 1 to 3. It should be noted that throughout the drawings, the dimensions of the shown components are not drawn to scale. In particular, the thicknesses of the layers are mostly exaggerated strongly.

As can been seen from Fig. 4, the process starts with depositing and structuring a highly reflective first contact metallization (p-contact metallization) 112 on a GaN layer stack 102, 103, 104 epitaxially grown on a sapphire substrate 128. As this is known, GaN PN double layers are particularly efficiently grown on sapphire. However, of course any other suitable substrate material may also be used according to the present disclosure. Also double heterostructure LEDs can be fabricated using the lateral electrical contacting according to the present disclosure. However, the following description will focus on a GaN homojunction LED.

In particular, an n-doped GaN layer 104 is deposited on the sapphire substrate 128. A p-doped GaN layer 102 is arranged on the n-doped GaN layer 104. The first contact metallization (p-contact metallization) 112 serves for electrically contacting the p-doped GaN layer 102.

In a next step (see Fig. 5), the p-doped GaN layer 102 and a part of the n-doped GaN layer 104 are patterned to define the footprint of the p-doped GaN layer 102 and thus of the PN junction. This patterning step is preferably performed by dry-etching, for instance by means of ion beam etching, plasma etching, or reactive ion etching (RIE). Other etching technologies may of course also be used. It should be noted that preferably less than half of the complete thickness of the n-doped GaN layer 104 is removed, leaving significantly more than half of the complete thickness of the n-doped GaN layer 104 for forming the later lateral contact area.

As shown in Fig. 6, now a passivation layer, which forms the inner passivation layer 114 of the micro LED 100, is deposited. The inner passivation layer 114 may comprise a single or multiple layers comprising for instance silicon nitride and/or silicon dioxide.

In a second etching step (see Fig. 7), the inner passivation layer 114 is structured together with the remaining n-doped GaN layer 104 to define the outline of the n-doped GaN layer 104. Most importantly, by means of this etching step, a lateral contact region 111 is defined which serves for circumferentially contacting the n-doped GaN layer 104. The lateral contact region 111 runs around the complete circumference of the n-doped GaN layer 104. Again, the etching is preferable performed by means of a dry etching process, e. g. ion beam etching, plasma etching, or reactive ion etching (RIE). Other etching technologies may of course also be used.

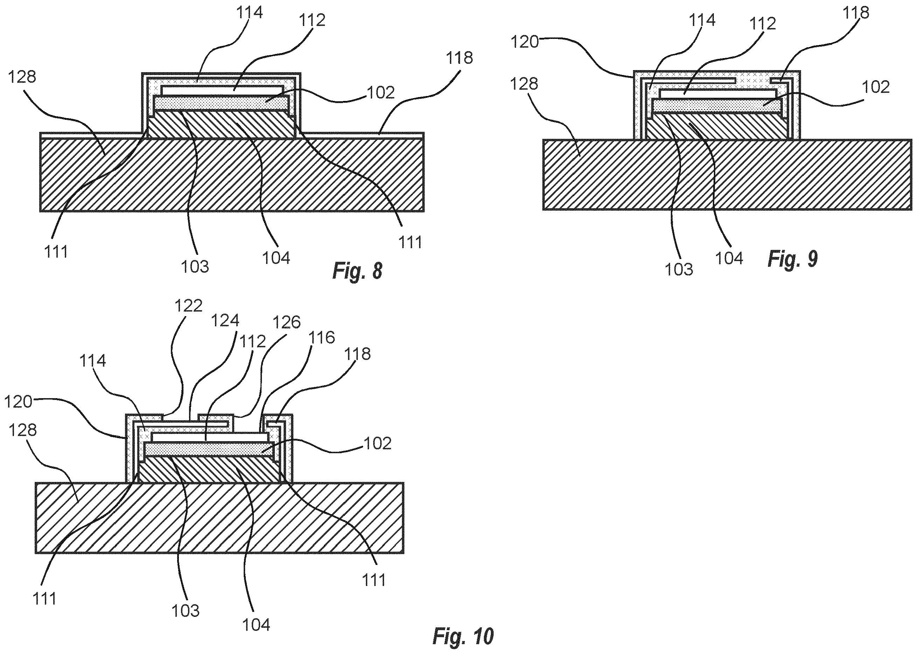

For electrically contacting the lateral contact region 111 of the n-doped GaN layer 104, a second contact metallization 118 is deposited, as shown in Fig. 8. The second contact metallization 118 is structured and for protection and electrical insulation, an outer passivation layer 120 is deposited and structured to define the final outline of the LED mesa (see Fig. 9). The second contact metallization 118 firstly provides an electrical connection to the lateral contact region 111 of the n-doped GaN layer 104, so that a particularly symmetric and low resistance contact is achieved. Secondly, the second contact metallization 118 is made of a material with a high reflectivity for the radiation generated by the micro LED 100, so that the second contact metallization 118 serves as a mirror layer reflecting all stray radiation ultimately towards the light-emitting facet 106.

In a last step, the outer passivation layer 120 is opened to form the first contact hole 126 and the second contact hole 122. Figure 10 shows the final LED mesa 100, still attached to the sapphire substrate 128.

One of the numerous application fields in which micro LEDs 100 according to the present disclosure, as described in the Fig. 1 to 10, may be utilized yielding significant synergetic advantages is the field of optical implants. For instance, an optogenetic neural probe, as proposed in Klein E, Ulrich CR, Paul O & Ruther P (2021) Stackable Wireless Controller of Single-sided µLED Arrays for Optogenetics in Freely Behaving Animals. In IEEE MEMS Conf. 2022, Tokyo, Japan, pp. 114-117, doi: 10.1109/MEMS51670.2022.9699683, can be improved significantly in terms of efficiency and safety when using arrays of micro LEDs 100 according to the present disclosure.

Klein E, Ulrich CR, Paul O & Ruther P (2021) Stackable Wireless Controller of Single-sided µLED Arrays for Optogenetics in Freely Behaving Animals. In IEEE MEMS Conf. 2022, Tokyo, Japan, pp. 114-117, doi: 10.1109/MEMS51670.2022.9699683, discloses a fully autonomous system comprising a wireless headstage and an array of micro LEDs integrated in a flexible neural probe for optogenetic experiments. The 49 micro LEDs (50x50 µm²) are arranged at a minimal pitch of only 100 µm. The micro LEDs of the optical neural probe are powered and controlled by a compact, modular headstage, which enables the simultaneous operation of multiple probes.

Generally, optogenetics enables the control of neural activity using light and offers clinical opportunities in restoring sensory functions (optical cochlear or retina implants), cardiology (pace maker and defibrillator) and other applications. Chronically implantable, wirelessly controlled optical probes with integrated light sources are a prerequisite for preclinical, optogenetic studies. In addition, probes with high spatial resolution are preferably flexible providing linear and 2D light patterns to optically stimulate neural tissue or muscle tissue of different cell density and functionality. In order to minimize the interference with freely behaving animals, energy-efficient light sources and controllers increasing the duration of uninterrupted in vivo experiments have to be developed.

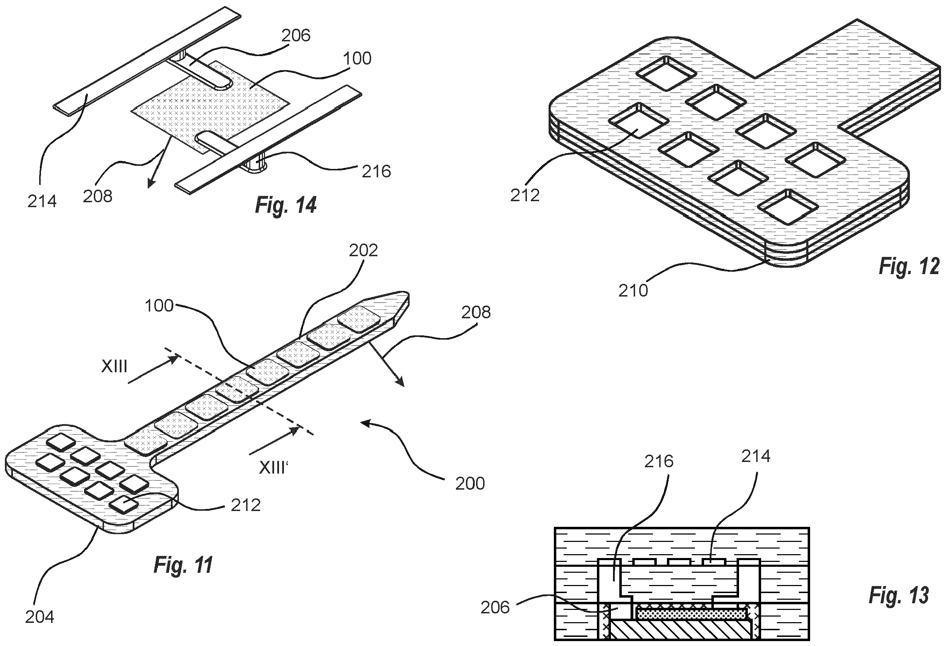

An optogenetic neural probe 200 according to the present disclosure (also called optical probe or optical implant) will now be described referring to the Fig. 11 to 14.

Figure 11 schematically shows the optical probe 200 of the publication Klein E, Ulrich CR, Paul O & Ruther P (2021) Stackable Wireless Controller of Single-sided µLED Arrays for Optogenetics in Freely Behaving Animals. In IEEE MEMS Conf. 2022, Tokyo, Japan pp. 114-117, doi: 10.1109/MEMS51670.2022.9699683, as modified according to the present disclosure. The optical probe 200 comprises a slender, flexible probe shank 202 with a linear array of micro LEDs 100 and a broader base 204 for the electrical interconnection to a flexible ribbon cable (not shown in the Figures) using bond pads 212.

As further detailed in Fig. 14, the micro LEDs 100 are electrically contacted from one side of the micro LED mesa 100 directly onto the first and second contact regions 116, 124 by means of conductive bridges 206, vias 216 and interconnection lines 214. As schematically indicated by the arrow 208 in Fig. 11 and Fig. 14, light is emitted towards the rear of the LED mesa 100, without shadowing by micro LED contacts and the respective contact metallization 112, 118.

The interconnecting lines ("shank wires" 214 in Fig. 13 and 14) of the probe shank 202 are sandwiched between three polymer layers 210 (Fig.12). The use of two metal layers forming bridges 206 (Fig. 13 and 14) and electroplated low-resistance feed lines 214 (Fig. 13 and 14) allows to effectively route the micro LEDs 100 in a multiplexed interconnection scheme without unnecessarily increasing the shank width. A further advantage of the electroplated feed lines is due to the fact that the contact pads 212 of the probe base 204 are mechanically more robust and located at the same level. Vias 216 (Fig. 13 and 14) provide the electrical connection between the various layers within the shank 202. The polymer may for instance comprise epoxy. However, any other suitable material may of course also be used.

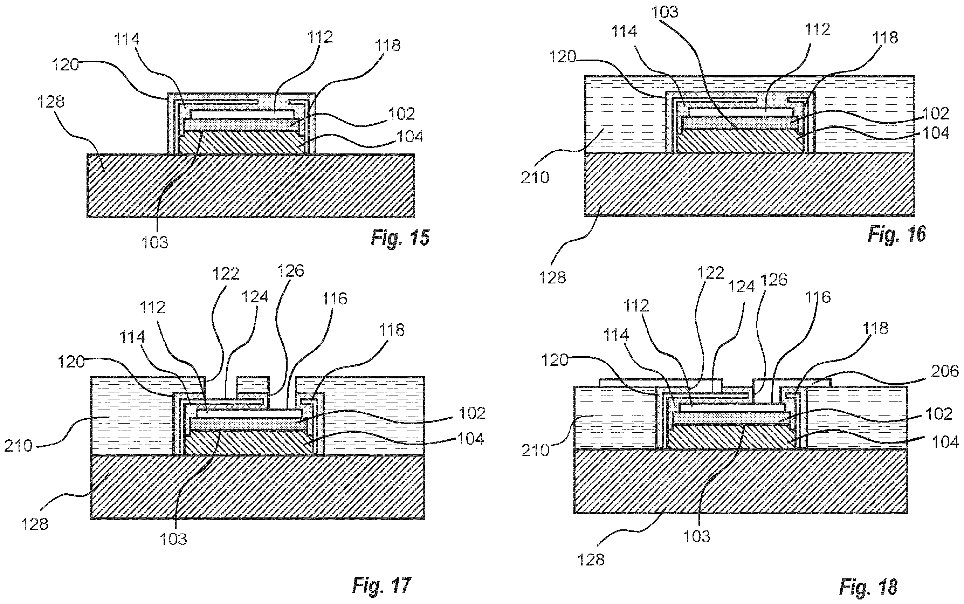

The fabrication process of the flexible micro LED-based optical probe 200 comprises the fabrication of the micro LEDs 100 in a manufacturing process, as described with reference to the Fig. 4 to 10, and a further modified polymer process including the metallization, as depicted in Fig. 15 to 22. Instead of the process step shown in Fig. 9, according to Fig. 15, the second contact metallization 118 is structured before the outer passivation layer 120 is deposited and structured (Fig. 15), in order to allow the forming of the second contact hole later.

As shown in Fig. 16, a first polymer layer 210 is applied onto the working substrate 128, covering the surface of the working substrate 128 and the outer passivation layer 120.

The process steps to realize the polymer probe substrate with embedded metallization are essentially as shown in Klein E, Ulrich CR, Paul O & Ruther P (2021) Stackable Wireless Controller of Single-sided µLED Arrays for Optogenetics in Freely Behaving Animals. In IEEE MEMS Conf. 2022, Tokyo, Japan, pp. 114-117, doi: 10.1109/MEMS51670.2022.9699683. This document shows the use of epoxy as a polymer and the example of epoxy will be explained in the following. However, of course any other suitable polymer may also be used according to the present disclosure. Further, the first to third polymer layers may also comprise different types of polymer and do not have to be fabricated from the same material. Moreover, the polymer layers may also be applied by means of other techniques than spin-coating, depending on the chosen polymer material.

According to this document, for substrate planarization a thicker epoxy layer (e. g. E301, Epoxy-Technologies, USA) is spin-coated onto the sapphire wafer 128 with the protruding micro LED mesas 100 (see Fig. 16).

In a next step, as shown in Fig. 17, the first polymer layer 210 and the outer passivation layer 120 are opened to provide access to the first contact metallization 112 and to the second contact metallization 118.

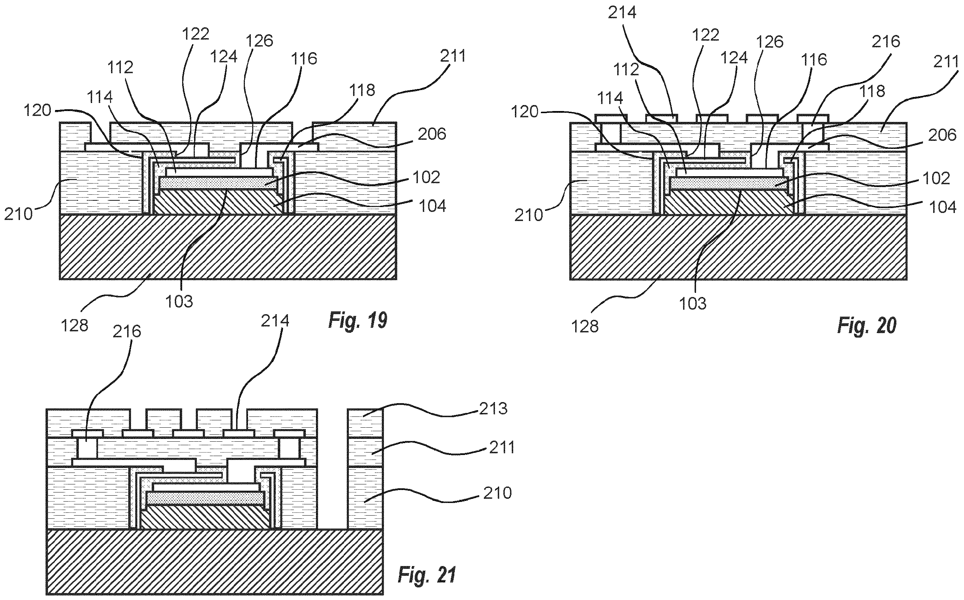

The micro LED contact regions 116, 124 are exposed as the first polymer layer 210 and the outer passivation layer 120 are opened before; by a maskless back-etch using reactive ion etching (RIE) the thickness of the first polymer layer 210 is reduced. Subsequently, the passivation layers 114, 120 at the contact sites 116, 124 are opened by RIE followed by the deposition and patterning of the bridge metal 206 using a lift-off technique (Fig. 18). Due to the relatively short length of these metal bridges 206, the thin-film metal provides a sufficiently low line resistance. After the p- and n-bridges 206 are defined on top of the planarized and patterned first epoxy layer 210, a second epoxy layer (second polymer layer 211 of Fig. 19) is spin-coated and equipped with vias 216 to the p- and n-bridges 206 patterned by RIE.

The epoxy insulates the n- and p-contact 116, 124 as well as the bridges 206 against the shank wiring deposited subsequently. The interconnection lines 214 interfacing the recessed vias are realized by lithography and gold electroplating to a thickness of ca. 2 µm.

In order to encapsulate the shank wiring 214, a third epoxy layer 213 is spin-coated onto the wafer. This layer is patterned by a final RIE process step to access the bond pads 212 at the probe base 204 and trench the three epoxy layers 210 down to the sapphire substrate 128 to define the final shape of the probe 200 (Fig. 21).

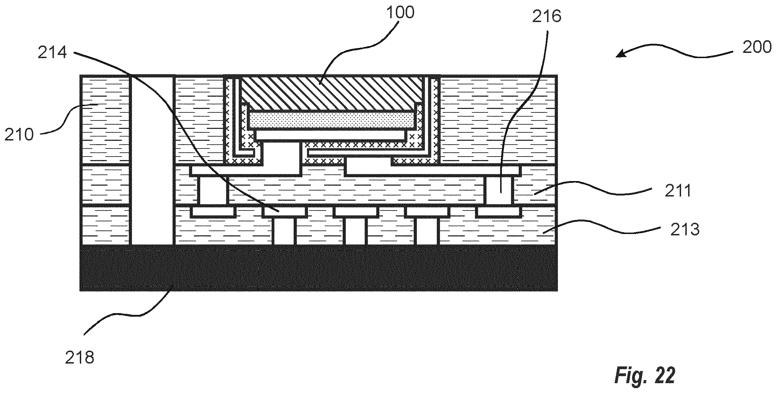

Ultimately, as shown in Fig. 22, the optical probes 200 are released from the sapphire wafer 128 onto a release tape 218 using laser lift-off, as described for instance in Klein E, Gossler C, Paul O & Ruther P (2018) High-density µLED-based optical cochlear implant with improved thermomechanical behavior. Front Neurosci 12: 659-659 (15pp).

In summary, at least the following key advantages can be achieved using the technologies according to the present disclosure: The highly resistive p-doped side of the micro LED is almost completely covered by the (highly) reflective metallization, e. g. based on silver, as described in Klein E, Gossler C, Paul O & Ruther P (2018) High-density µLED-based optical cochlear implant with improved thermomechanical behavior. Front Neurosci 12: 659-659 (15pp); the slight overlap of the micro LED with respect to the p-contact is defined by the lithographic accuracy.

The n-contact exploits the entire circumference of the micro LED mesa 100 (height e. g. ca. 3 µm, length defined by the footprint, here 4×50 µm = 200 µm, resulting in a contact area of 600 µm²). This leads to a low contact resistance and a homogenized and symmetric current flow in the micro LED mesa avoiding the current crowding at the smaller n-contact which is characteristic for the single-sided approach of known micro LEDs.

The n-contact is designed such that it covers the entire micro LED mesa except for the through via required for the wiring interfacing the p-contact. In this way, stray light that would normally be exiting the micro LED mesa through its sidewalls is reflected back increasing the chance that it exits the micro LED through the n-doped, active (bottom) side.

The overall active area per micro LED is increased in comparison to the conventional single-sided surface contacts. This effect is especially strong for micro LEDs with small area, e. g. less than 100×100µm².

**Reference Numerals**

| **Reference Numeral** | **Description** |
|---|---|
| 100 | Micro light-emitting diode (LED) |
| 102 | First semiconductor region; p-GaN |
| 103 | Optically active region |
| 104 | Second semiconductor region; n-GaN |
| 106 | Active side; light-emitting facet |
| 108 | Non-active side; non-emitting side |
| 110 | Lateral region |
| 111 | Lateral contact region |
| 112 | First contact metallization |
| 114 | Inner passivation layer |
| 116 | First contact region |
| 118 | Second contact metallization |
| 120 | Outer passivation layer |
| 122 | Second contact hole |
| 124 | Second contact region |
| 126 | First contact hole |
| 128 | Substrate; working substrate |
| 200 | Neural implant; optogenetic neural probe |
| 202 | Probe shank |
| 204 | Probe base |
| 206 | Bridge |
| 208 | Direction of emitted light |
| 210 | First polymer layer; carrier layer |
| 211 | Second polymer layer |
| 212 | Bond pads |
| 213 | Third polymer layer |
| 214 | Shank wires; interconnection lines |
| 216 | Via |
| 218 | Release tape |

## Claims

1. Micro LED (100) comprising an active side (106), from which radiation is emitted in operation, and a non-emitting side (108), which extends opposite to the active side (106), and a lateral region (110) which surrounds the micro LED (100) between the active side (106) and the non-emitting side (108), the micro LED (100) further comprising:
a first semiconductor region (102),
a second semiconductor region (104),
an optically active region (103) arranged between the first and second semiconductor regions (102, 104),
a first contact metallization (112) for electrically contacting the first semiconductor region (102), and
a second contact metallization (118) for electrically contacting the second semiconductor region (104),
wherein the second contact metallization (118) is electrically connected to the second semiconductor region (104) in a lateral contact region (111) which is part of the lateral region (110).

2. Micro LED (100) according to claim 1, wherein the first semiconductor region (102) is a p-doped region, and the second semiconductor region (104) is an n-doped region.

3. Micro LED (100) according to claim 2, wherein the first contact metallization (112) comprises a planar electrode arranged on the non-emitting side (108) for electrically contacting the p-doped region (102).

4. Micro LED (100) according to claim 2 or 3, wherein the n-doped region (104) comprises n-doped GaN, and the p-doped region (102) comprises p-doped GaN.

5. Micro LED (100) according to one of the preceding claims, wherein the second contact metallization (118) at least partly covers the non-emitting side (108) and the lateral region (110).

6. Micro LED (100) according to one of the preceding claims, wherein the first contact metallization (112) and/or the second contact metallization (118) comprise silver or a silver alloy.

7. Micro LED according to one of the preceding claims, further comprising an outer passivation layer (120) that covers the non-emitting side (108) and the lateral region (110), the outer passivation layer (120) being provided with at least one first contact hole (126) through which the first contact metallization (112) is accessible, and with at least one second contact hole (124) through which the second contact metallization (118) is accessible.

8. Neural implant (200) comprising at least one micro LED (100) according to one of the preceding claims, the neural implant (200) further comprising an at least two-layered carrier substrate (210), comprising a carrier layer and a first flexible polymer layer.

9. Method of fabricating a micro LED (100), the method comprising the following steps:
depositing an optically active layer stack (102, 103, 104) on a working substrate (128), the optically active layer stack (102, 103, 104) comprising a first semiconductor region (102), a second semiconductor region (104) which is in contact with the working substrate (128), and an optically active region (103) arranged between the first and second semiconductor regions (102, 104);
depositing and structuring a first contact metallization (112) to contact the first semiconductor region (102);
structuring the first semiconductor region (102) and at least a part of the second semiconductor region (104) to expose the sidewall of the first semiconductor region (102), the sidewall of the optically active region (103), and the partial sidewall of the second semiconductor region (104);
depositing an inner passivation layer (114) so that it covers the first contact metallization (112), the sidewall of the first semiconductor region (102), the sidewall of the optically active region (103), and the partial sidewall of the second semiconductor region (104);
performing an etching step, which exposes a lateral contact region (111) of the second semiconductor region (104), the lateral contact region (111) not being covered by the inner passivation layer;
depositing and structuring a second contact metallization (118), so that the second contact metallization (118) is electrically connected to the second semiconductor region (104) in a lateral region (110) comprising the lateral contact region (111).

10. Method according to claim 9, wherein the first semiconductor region (102) is a p-doped region, and the second semiconductor region (104) is an n-doped region, which are in contact with the optically active region (103) that is sandwiched in-between and operable to emit light.

11. Method according to claim 9 or 10, further comprising the step of depositing an outer passivation layer (120) onto the micro LED (100), so that the non-emitting side (108) and the lateral region (110) are at least partly covered, the outer passivation layer (120) being provided with at least one first contact hole (126) through which the first contact metallization (112) is accessible, and with at least one second contact hole (122) through which the second contact metallization (118) is accessible.

12. Method according to claim 10 or 11, wherein the n-doped region (104) comprises n-doped GaN, and the p-doped region (102) comprises p-doped GaN.

13. Method according to one of the claims 9 to 12, wherein the first contact metallization (112) and/or the second contact metallization (118) has in at least a part of a spectral range between 300 nm and 1200 nm a reflectivity of more than 98 % and a transmittance of less than 2 %.

14. Method according to one of the claims 9 to 13, wherein the first contact metallization (112) and/or the second contact metallization (118) comprise silver or a silver alloy.

15. Method according to one of the claims 9 to 14, wherein the inner passivation layer (114) and/or the outer passivation layer (120) comprise a silicon nitride and/or a silicon dioxide layer.
